# EUROPEAN PATENT SPECIFICATION

(11) **EP 3 178 474 B1**
(45) Date of publication and mention of the grant of the patent: **18.03.2020**
(21) Application number: 16202982.1
(22) Date of filing: 22.03.2012
(51) Int. Cl.: A61K 9/48

(54) **PHARMACEUTICAL FORMULATION COMPRISING INOSITOL**
PHARMAZEUTISCHE FORMULIERUNG MIT INOSITOL
FORMULATION PHARMACEUTIQUE COMPRENANT DE L'INOSITOL

(30) Priority: 22.03.2011 IT MI20110445
(43) Date of publication of application: 14.06.2017
(62) Divisional of application: 12160674.3
(73) Proprietor: LO. LI. Pharma S.r.l., 00156 Roma (IT)
(72) Inventor: UNFER, Vittorio, 00155 Rome (IT)
(74) Representative: Grünecker Patent- und Rechtsanwälte PartG mbB

(56) References cited:
- US-A1- 2007 243 211
- JOHN E NESTLER: "OVULATORY AND METABOLIC EFFECTS OF D-CHIRO-INOSITOL IN THE POLYCYSTIC OVARY SYNDROME", NEW ENGLAND JOURNAL OF MEDICINE, THE, MASSACHUSETTS MEDICAL SOCIETY, WALTHAM, MA, US, 29 April 1999 (1999-04-29), pages 1314-1320, XP007906382, ISSN: 0028-4793
- P RIZZO ET AL: "Effect of the treatment with myo-inositol plus folic acid plus melatonin in comparison with a treatment with myo-inositol plus folic acid on oocyte quality and pregnancy outcome in IVF cycles. A prospective, clinical trial", EUROPEAN REVIEW FOR MEDICAL AND PHARMACOLOGICAL SCIENCES, vol. 14, 10 June 2010 (2010-06-10), pages 555-561, XP55241120,

## Description

### FIELD OF THE INVENTION

The present invention relates to a pharmaceutical composition comprising myo-inositol.

### PRIOR ART

Inositol, a chemical compound of formula C₆H₁₂O₆, is a hydrocarbon having a structure different to that of conventional sugars. It exists as nine possible isomers, of which the most important form, widely present in nature, is cis-1, 2, 3, 5-trans-4, 6-cyclohexanehexol, or myo-inositol.

Apart from myo-inositol, the other isomers that exist, even if only in minimal quantities, are scyllo-, muco-, D-chiro-, L-chiro-, neo-, allo-, epi- and cis-inositol.

Inositol has a molecular formula identical to that of glucose, although it differs in molecular structure. It is synthesized by the organism directly from glucose-6-phosphate, and for this reason it is frequently marked out as a pseudo-vitamin, forming part of the B group and called vitamin B8.

Inositol plays a fundamental role in the secondary messengers within the cells, in form of inositol phosphate, or as phosphatidyl-inositol (PI) or phosphatidyl-inositol phosphate (PIP).

Myo-inositol, in particular, participates in important processes, such as morphogenesis and cytogenesis, lipid synthesis, the constitution of the cell membrane, and cell growth (Berridge, M.J. "Inositol lipids and cell proliferation", Biochim Biophys Acta 1987; 907: 33-45; Downes C.P. "The cellular function of myo-inositol", Biochem Soc Trans 1989; 17:259-68). Scientific studies have demonstrated that myo-inositol is co-involved as a precursor in the synthesis of phospho-inosides and constitutes the system of transduction of signals of phosphatidyl-inositol (PtdIns), which is known to be co-involved in the regulation of various cellular functions, including gametogenesis, fertilization, cell proliferation and development, secretion, contraction and neural activity (Berridge, M.J., Irvine R.F. "Inositol phosphate and cell signalling", Nature 1989; 306: 197-205; Divecha N., Irvine R.F. "Phospholipid signalling", Cell 1995; 80:269-78; Herbert M., Gillespie, J.I., Murdoch, A.P. "Development of calcium signaling mechanisms during maturation of human oocytes" Mol Hum Reprod 1997; 3: 965-73; Berridge, M.J., Downes, C.P., Hanley, M.R. "Neural developmental actions of lithium: a unifying hypothesis", Cell 1989; 59:411-9).

Since the work of Nestler et al. in 2000 (Nestler, J.E., Jakubowicz, D.J., luorno, M.J. "Role of inositol phosphoglycan mediators of insulin action in the polycystic ovary syndrome", J Pediatr Endocrinol Metab. 2000; 13 Suppl 5:1295-8), more and more scientific evidence has accumulated that supports the physiological and therapeutic role of inositol, particularly in disorders linked to ovarian polycystosis.

Polycystic ovary syndrome or ovarian polycystosis, (PCOS), is a complex and heterogeneous disorder that affects 6-10% of women of reproductive age (Diamanti-Kandarakis E., Argyrakopoulou G., Economou F., Kandaraki E., Koutsilieris M. "Defects in insulin signaling pathways in ovarian steroidogenesis and other tissues in polycystic ovary syndrome (PCOS)", J Steroid Biochem Mol Biol 2008; 109: 242-6), and is the principal cause of infertility (Dunaif A. "Insulin resistance and the polycystic ovary syndrome: mechanism and implications for pathogenesis", Endocr Rev 1997; 18: 774-800). It is characterized principally by chronic anovulation, hyperandrogenism, an altered LH/FSH ratio (> 2/3:1) and by characteristic structure of the polycystic ovary, verifiable by echographic analysis

It has now been widely demonstrated that insulin resistance is intrinsically linked to polycystic ovary syndrome. Indeed, insulin resistance is present in 50-70% of women with PCOS, independently of whether they are obese or of normal weight. This disorder is considered the major factor in the pathogenesis of the syndrome ((Dunaif A. "Insulin resistance and the polycystic ovary syndrome: mechanism and implications for pathogenesis", Endocr Rev 1997; 18: 774-800; Legro R.S., Gnatuk C.L., Kunselman A.R., Dunaif A. "Changes in glucose tolerance over time in women with polycystic ovary syndrome: a controlled study", J Clin Endocrinol Metab 2005; 90: 3236-42). Women affected with PCOS are often obese, and this contributes to the development of insulin resistance. It is well known that insulin resistance frequently spontaneously develops in the direction of the onset of compensatory hyperinsulinemia, which leads to the hyperandrogenism typical of the syndrome (Poretsky L., Cataldo N., Rosenwaks Z., Guidice L. "The insulin-related ovarian regulatory system in health and disease" Endocr Rev 1999; 20: 532-82). The excess of androgen hormones results in menstrual irregularity, the development of ovarian cysts, hirsutism, and other disorders related to those mentioned. In these women with PCOS, insulin resistance can furthermore increase the risk of developing glucose intolerance, type 2 diabetes mellitus, hypertension, dyslipidemia and cardiovascular problems (Legro R.S., Gnatuk C.L., Kunselman A.R., Dunaif A. "Changes in glucose tolerance over time in women with polycystic ovary syndrome: a controlled study", J Clin Endocrinol Metab 2005; 90: 3236-42); Maitra A., Pingle R.R., Menon P.S., Naik V., Gokral J.S., Meherji P.K. "Dyslipidemia with particular regard to apolipoprotein profile in association with polycystic ovary syndrome: a study among Indian women" Int. J. Fertil Womens Med. 2001; 46: 271-7).

Hyperinsulinemia and hyperandrogenism are therefore the two principal characteristic factors of polycystic ovary syndrome, even if their cause-and-effect relationship is still the subject of debate (Dunaif A. "Changes in glucose tolerance over time in women with polycystic ovary syndrome: a controlled study", J Clin Endocrinol Metab 2005; 90: 3236-42; Bremer A.A., Miller W.L. "The serine phosphorylation hypothesis of polycystic ovary syndrome: a unifying mechanism for hyperandrogenemia and insulin resistance" Fertil Steril 2008; 89: 1039-48). However, much scientific evidence suggests that hyperinsulinemia is the primary factor contributing to ovarian hyperandrogenism. The pharmacologically achieved lowering of the insulin levels results in improvement of the hyperinsulinemia and hyperandrogenism, and restoration of normal ovarian function in women with PCOS (Dunaif A. "Changes in glucose tolerance over time in women with polycystic ovary syndrome: a controlled study", J Clin Endocrinol Metab 2005; 90: 3236-42; Bremer A.A., Miller W.L. "The serine phosphorylation hypothesis of polycystic ovary syndrome: a unifying mechanism for hyperandrogenemia and insulin resistance" Fertil Steril 2008; 89: 1039-48).

PCOS women who are obese, and those of normal weight, present insulin resistance independently of the fat mass (Dunaif A., Segal K.R., Futterweit W., Dobrjansky A. "Profound peripheral insulin resistance, independent of obesity, in polycystic ovary syndrome" Diabetes 1989; 38: 1165-1174), and the scientific evidence suggests that a deficiency of this particular inositol phosphoglycan, containing D-chiro-inositol, may contribute to insulin resistance in individuals with glucose intolerance or type 2 diabetes (Kennington A.S., Hill C.R., Craig J., et al. "Low urinary chiro-inositol excretion in non-insulin-dependent diabetes mellitus" N.Engl. J. Med. 1990; 323: 373-378). Indeed, administration of substances having insulin-sensitizing activity, such as D-chiro-inositol (Iuorno M.J., Jakubowicz D.J., Baillargeon J.P., Dillon P., Gunn R.D., Allan G., Nestler J.E. "Effects of d-chiro-inositol in lean women the polycystic ovary syndrome" Endocr Pract 2002; 8: 417-423; Nestler J.E., Jakubowicz D.J., Reamer P., Gunn R.D., Allan G. "Ovulatory and metabolic effects of d-chiro-inositol in the polycystic ovary syndrome" N.Engl. J. Med. 1999; 340: 1314-1320) to PCOS women who are obese or those of normal weight increases the frequency of ovulation and reduces the levels of circulating androgens. In support of this hypothesis, some studies have demonstrated that oral administration of D-chiro-inositol improved glucose tolerance by reducing insulin levels in women with PCOS, whether of normal weight (Iuorno M.J., Jakubowicz D.J., Baillargeon J.P., Dillon P., Gunn R.D., Allan G., Nestler J.E. "Effects of d-chiro-inositol in lean women with the polycystic ovary syndrome" Endocr Pract 2002; 8: 417-423), or obese (Nestler J.E., Jakubowicz D.J., Reamer P., Gunn R.D., Allan G. "Ovulatory and metabolic effects of d-chiro-inositol in the polycystic ovary syndrome" N.Engl. J. Med. 1999; 340: 1314-1320). In such patients, it also reduced the levels of androgens, improved ovarian function, and led to a reduction in testosterone levels in the blood and to an improvement in metabolic parameters, such as arterial blood pressure and triglyceride levels in women with PCOS (Genazzani A.D., Lanzoni C., Ricchieri F., Jasonni V.M. "Myo-inositol administration positively affects hyperinsulinemia and hormonal parameters in overweight patients with polycystic ovary syndrome" Gynecol Endocrinol 2008; 24(3): 139-44).

Inositol is commonly used in clinical practice for treating the ovarian polycytosis syndrome. Various products containing inositol are commercially available, the pharmaceutical formulations of which are granulate or tablets.

During the research work performed on inositol, it was discovered that light, humidity, temperature, contact with oxygen, the pH, the production process, the presence of excipients, etc are degrading factors that can affect the inositol titer and the presence of yeasts and moulds.

### SUMMARY OF THE INVENTION

The aim of the present invention is to provide an easy-to-use formulation of myoinositol for oral use, wherein the inositol titer is stable for prolonged periods.

It is also the objective of the present invention to provide a formulation of myoinositol for oral use that enables a plasma inositol concentration to be achieved which is higher than that obtained with the forms currently available.

Another objective of the present invention is to provide a method of manufacturing a formulation of myoinositol for oral use that is both of stable inositol titer and easy to use.

One objective of the present invention is to provide a composition for the treatment of polycystic ovary syndrome.

The scope of the invention is defined by the claims. Any references in the description to methods of treatment refer to the products of the present invention for use in a method for treatment.

### BRIEF DESCRIPTION OF THE DRAWING

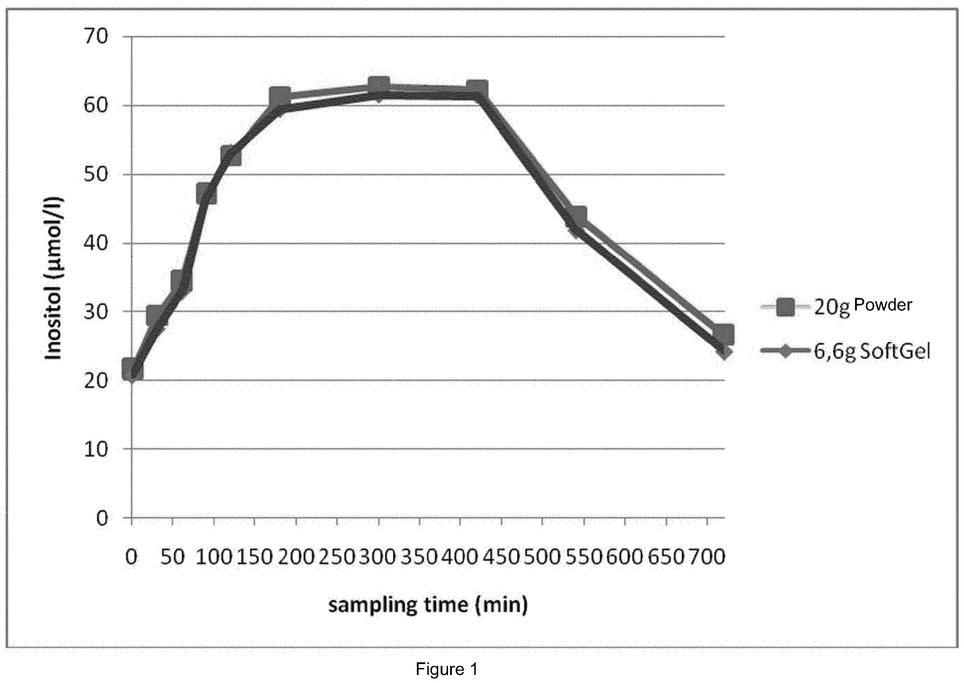

Figure 1 is a diagram showing the increase of the inositol concentration in plasma when inositol is administered in the form of powder or soft gel respectively.

### DETAILED DESCRIPTION OF THE INVENTION

These aims, and others which will be described below, have been achieved by means of a a soft capsule as defined in claim 1 or claim 2.

The aims of the present invention have also been achieved by means of said composition for use in the treatment and/or prevention of polycystic ovary syndrome, insulin resistance, hyperinsulinemia, hypoglycemia, hyperandrogenism, metabolic syndrome, dyslipidemia, type 2 diabetes mellitus and cardiovascular/ cerebrovascular diseases; it is also used in Medically Assisted Procreation (MAP) therapies in order to improve oocyte quality, and to optimize ovarian hyperstimulation protocols; in particular to prevent ovarian hyperstimulation syndrome. Further beneficial effects have been observed on the classical symptoms of the menopause, such as: irritability, hypertension, osteoporosis, dyslipidemia, weight gain, hot flushes and aging of the skin.

The aims of the present invention have also been achieved by means of a process for the manufacture of said soft capsule comprising the dissolution, suspension or dispersion of myoinositol and of at least one excipient and/or plastifier in a vehicle comprising glycerol, gelatin or mixtures thereof.

Within the scope of the present invention, semi-liquid phase means the various types of suspension with which it is possible to formulate inositol and any constituents present in the composition.

Within the scope of the present invention, "softgel" is used to mean a dosage form consisting of a gelatin-based shell that encloses a liquid filling, wherein the shell comprises a combination of gelatin, water, opacifier and plastifier such as glycerin and/or sorbitol. The liquid filling of the soft capsule is as defined in claims 1 or 2, and is enclosed by a shell as defined above, forming what is known as a "softgel pearl".

Surprisingly, it was found that the soft capsules forms for oral administration, obtained according to the present invention, are clearly less sensitive to the various degrading influences described above for the known pharmaceutical forms, as can be noted from the data presented in the table.

**Table. Stability data inositol titration.**

| Stability | Pharmaceutical form powder | Pharmaceutical form softgel |
|---|---|---|
| Time 0 | 120 | 120 |
| 6 months | 115 | 119 |
| 12 months | 111 | 119 |

In particular, the present invention comprises myoinositol-based pharmaceutical compositions in soft capsules having uniform matrices which, as well as being free from micro-contaminations that can further auto-catalyse decomposition, determine additional advantages such as, for example, the elevated and more immediate bioavailability of the active principle within the gastrointestinal environment.

It was discovered in pilot studies that the pharmacokinetic parameters are surprisingly superior to those obtained with known formulations. In particular, one third of the doses of inositol, which was administered as soft gelatin capsules, induces an increase in the plasma concentration comparable to that induced by the powder, as can be seen in Fig. 1.

The soft capsule according to the present invention is a dosage form consisting of a gelatin-based shell and a filling of said shell, comprising said one solution, suspension or dispersion of myoinositol in a vehicle comprising gelatin, glycerol, ethanol or mixtures thereof.

In said composition, the dosage form is a soft capsule.

In the composition according to the present invention, said shell is preferably coated with an outer coating that allows the release of inositol in the small intestine. Such a coating can be produced on the basis of the prior art in such a way as to break up substantially within the region of the small intestine, the principal location of inositol absorption.

In the soft capsule according to the present invention, said shell is preferably coated with an outer coating that facilitates ingestion.

In a preferred embodiment, the composition according to the present invention is in the form of a softgel pearl of uniform matrix, comprising glycerol and myoinositol.

Furthermore, the hardness of the composition according to the present invention in the form of swallowable soft or softgel capsules of uniform matrix can be controlled on the basis of the capsule type or the uniform matrix of the swallowable softgel, which capsule type or uniform matrix are to be obtained by means of known plastifiers pharmacologically accepted for capsules, such as, for example, polyhydroxy alcohols, preferably glycerol, 1,2-propylene glycol, sorbitol solutions, etc.

In the case wherein the composition of the invention consists of softgel of uniform matrix, comprising both myoinositol and the possible excipients and/or plastifiers, such a structure allows a rapid release of the contents, from the envelope or from the matrix respectively, and therefore having a rapid release of the active principle, which has already been dissolved and/or dispersed.

The materials used to obtain the swallowable soft or softgel capsules of uniform matrix according to the present invention are common gelatins of so-called type A (elastic and soft capsule consisting of a shell of gelatin material containing a liquid or semi-liquid phase including, within it, the principal dissolved in gelatin, and/or glycerol), and type B (swallowable softgel pearl of uniform matrix composed of glycerol and of the principle) used within the pharmaceutical field, or methylcellulose, hydroxypropylmethylcellulose, calcium alginate, or other suitable materials of the pharmaceutical prior art that can also be used for the same purposes.

Said composition in the form of swallowable softgel pearls preferably also comprises type A or type B gelatin.

The composition of the invention preferably also comprises at least one active principle different from myoinositol, for example, folic acid, cocoa polyphenols, genistein, L-arginine, vitamin E, selenium, N-acetylcysteine, and melatonin.

Further, optional common constituents of the soft or swallowable softgel capsules of uniform matrix, according to the present invention, are water and preservatives (such as antibacterials, antifungals, etc.), according to requirements.

The facultative excipients which may be used in preparing the uniform matrices of the swallowable softgels comprise the pharmacologically accepted constituents, such as, for example, solid additives as thickeners, which may become dissolved or dispersed in the liquid vehicle before or during gelification of the matrix, and/or preservatives.

As non-limiting examples of gelatin, type A or B are preferable, while plastifiers may be added to modify the elasticity of the softgel in cases wherein the vehicles and/or the excipients previously referred to are not sufficient to achieve the desired result.

In particular, even in the case of the swallowable softgels of uniform matrix, the substances which provide multiple functions, for example glycerol (as a vehicle and/or plastifier) are especially preferred.

The composition according to the present invention preferably also comprises a plastifier.

More preferably, in said composition the plastifier is selected from glycerol, 1,2-polypropylene glycol, a sorbitol solution and mixtures thereof.

In the composition according to the invention, said vehicle preferably further comprises ethanol and glycerol.

Myoinositol is preferably present in the composition according to the invention in a quantity between 100 mg and 2 g. It was surprisingly found that the compositions of the present invention can contain relatively elevated quantities of myoinositol without stability problems, thus enabling the administration of high doses to the patient with only one dose; administration in this pharmaceutical form furthermore facilitates the absorption of inositol.

In one aspect, the present invention relates to a process for manufacturing said soft capsule comprising the dissolution, suspension or dispersion of myoinositol or of at least one isomer thereof, and of at least one excipient and/or plastifier, in a vehicle comprising glycerol, gelatin or mixtures thereof.

A further advantage of the composition of the invention in the form of swallowable softgel of uniform matrix derives from the fact that the pearl can be divided, at least in the case wherein they are not provided with enteric or similar coatings, on the recommendation of the doctor on behalf of the patient himself or herself, to allow further refinement of the daily dose beyond the units of the standard dose delivered by the pharmaceutical product. Moreover, in all the cases wherein solutions of active principles are used to obtain the gel matrix, the production of perfectly homogenous dosages is rendered especially easy.

In another aspect, the composition according to the invention is used in the treatment and/or prevention of pathologies of polycystic ovary syndrome, insulin resistance, hyperinsulinemia, hyperglycemia, hyperandrogenism, metabolic syndrome, dyslipidemia, type 2 diabetes mellitus and cardiovascular/cerebrovascular diseases; it is also used in Medically Assisted Procreation (MAP) therapies in order to improve oocyte quality, and to optimize ovarian hyperstimulation protocols; in particular to prevent the ovarian hyperstimulation syndrome.

Further beneficial effects have been observed on the classical symptoms of the menopause, such as: irritability, hypertension, osteoporosis, dyslipidemia, weight gain, hot flushes and aging of the skin.

## Claims

1. A soft capsule consisting of a gelatin-based shell and a filling of said shell,
said filling comprising a solution, suspension or dispersion of active principles, said active principles being:
• inositol; and
• at least one of folic acid, cocoa polyphenols, genistein, L-arginine, vitamin E, selenium, N-acetylcysteine and melatonin;
in a vehicle comprising gelatin, glycerol, ethanol or mixtures thereof,
wherein said inositol is myo-inositol.

2. A soft capsule consisting of a gelatin-based shell and a filling of said shell,
said filling comprising a solution, suspension or dispersion of inositol,
in a vehicle comprising gelatin, glycerol, ethanol or mixtures thereof, wherein the shell is optionally coated with an outer coating that allows the release of inositol in the small intestine, and wherein said inositol is myo-inositol.

3. The soft capsule according to any one of the preceding claims, wherein said shell is coated with an outer coating which facilitates ingestion.

4. The soft capsule according to any one of the preceding claims, wherein said softgel capsule is swallowable.

5. The soft capsule according to any one of the preceding claims, wherein said shell further comprises a plastifier.

6. The soft capsule according to claim 5, wherein said plastifier is selected from the group consisting of polyhydroxy alcohols, glycerol, 1,2-propylene glycol and sorbitol solutions.

7. The soft capsule according to any one of the preceding claims, for use in the treatment and/or prevention of polycystic ovary syndrome, insulin resistance, hyperinsulinemia, hyperglycemia, hyperandrogenism, metabolic syndrome, dyslipidemia, type 2 diabetes mellitus and cardiovascular/cerebrovascular diseases, in Medically Assisted Procreation (MAP) therapies in order to improve oocyte quality, and to optimize ovarian hyperstimulation protocols; in particular to prevent ovarian hyperstimulation syndrome, irritability, hypertension, osteoporosis, dyslipidemia, weight gain, hot flushes and aging of the skin.

## Patentansprüche

1. Weichkapsel, bestehend aus einer gelatinebasierten Hülle und einer Füllung von besagter Hülle,
wobei besagte Füllung eine Lösung, Suspension oder Dispersion von Wirkstoffen umfasst, wobei besagte Wirkstoffe sind:
- Inositol; und
- mindestens eines von Folsäure, Kakao-Polyphenolen, Genistein, L-Arginin, Vitamin E, Selen, N-Acetylcystein und Melatonin;
in einem Vehikel, das Gelatine, Glycerin, Ethanol oder Mischungen davon umfasst,
wobei besagtes Inositol Myo-Inositol ist.

2. Weichkapsel, bestehend aus einer gelatinebasierten Hülle und einer Füllung von besagter Hülle,
wobei besagte Füllung eine Lösung, Suspension oder Dispersion von Inositol umfasst,
in einem Vehikel, das Gelatine, Glycerin, Ethanol oder Mischungen davon umfasst, wobei die Hülle optional mit einer äußeren Beschichtung beschichtet ist, die die Freisetzung von Inositol im Dünndarm ermöglicht, und wobei besagtes Inositol Myo-Inositol ist.

3. Die Weichkapsel nach irgendeinem der vorstehenden Ansprüche, wobei besagte Hülle mit einer äußeren Beschichtung beschichtet ist, die die Einnahme erleichtert.

4. Die Weichkapsel nach irgendeinem der vorstehenden Ansprüche, wobei besagte Weichgelkapsel schluckbar ist.

5. Die Weichkapsel nach irgendeinem der vorstehenden Ansprüche, wobei besagte Hülle weiter einen Weichmacher umfasst.

6. Die Weichkapsel nach Anspruch 5, wobei besagter Weichmacher aus der Gruppe ausgewählt ist, bestehend aus Polyhydroxyalkoholen, Glycerin, 1,2-Propylenglykol und Sorbitollösungen.

7. Die Weichkapsel nach irgendeinem der vorstehenden Ansprüche zur Verwendung bei der Behandlung und/oder Prävention von polyzystischem Ovarialsyndrom, Insulinresistenz, Hyperinsulinämie, Hyperglykämie, Hyperandrogenismus, metabolischem Syndrom, Dyslipidämie, Typ-2-Diabetes Mellitus und kardiovaskulären/zerebrovaskulären Erkrankungen, bei Therapien der medizinisch unterstützten Fortpflanzung (MAP) zur Verbesserung der Eizellqualität und zur Optimierung von Protokollen zur ovariellen Hyperstimulation; insbesondere zur Vorbeugung des ovariellen Überstimulationssyndroms, Reizbarkeit, Bluthochdruck, Osteoporose, Dyslipidämie, Gewichtszunahme, Hitzewallungen und Hautalterung.

## Revendications

1. Capsule molle constituée d'une enveloppe à base de gélatine et d'un remplissage de ladite enveloppe,
ledit remplissage comprenant une solution, une suspension ou une dispersion de principes actifs, lesdits principes actifs étant :
• l'inositol ; et
• au moins l'un de l'acide folique, des polyphénols de cacao, de la génistéine, de la L-arginine, de la vitamine E, du sélénium, de la N-acétylcystéine et de la mélatonine ;
dans un véhicule comprenant de la gélatine, du glycérol, de l'éthanol ou leurs mélanges, où ledit inositol est le myo-inositol.

2. Capsule molle constituée d'une enveloppe à base de gélatine et d'un remplissage de ladite enveloppe,
ledit remplissage comprenant une solution, une suspension ou une dispersion d'inositol,
dans un véhicule comprenant de la gélatine, du glycérol, de l'éthanol ou leurs mélanges, où l'enveloppe est éventuellement enrobée d'un enrobage externe qui permet la libération d'inositol dans l'intestin grêle, et où ledit inositol est le myo-inositol.

3. Capsule molle selon l'une quelconque des revendications précédentes, dans laquelle ladite enveloppe est enrobée d'un enrobage externe qui facilite l'ingestion.

4. Capsule molle selon l'une quelconque des revendications précédentes, dans laquelle ladite capsule de gel mou peut être avalée.

5. Capsule molle selon l'une quelconque des revendications précédentes, dans laquelle ladite enveloppe comprend en outre un agent plastifiant.

6. Capsule molle selon la revendication 5, dans laquelle ledit agent plastifiant est sélectionné dans le groupe constitué des poly(alcools hydroxy), du glycérol, du 1,2-propylène glycol et des solutions de sorbitol.

7. Capsule molle selon l'une quelconque des revendications précédentes, pour l'utilisation dans le traitement et/ou la prévention du syndrome de l'ovaire polykystique, de la résistance à l'insuline, de l'hyper-insulinémie, de l'hyperglycémie, de l'hyperandrogénisme, du syndrome métabolique, de la dyslipidémie, du diabète sucré de type 2 et des maladies cardiovasculaires/cérébrovasculaires, dans les thérapies de procréation médicalement assistée (MAP) afin d'améliorer la qualité des oocytes, et d'optimiser les protocoles d'hyperstimulation ovarienne ; en particulier pour prévenir le syndrome d'hyperstimulation ovarienne, l'irritabilité, l'hypertension, l'ostéoporose, la dyslipidémie, le gain de poids, les bouffées de chaleur et le vieillissement de la peau.
